(19) Europäisches Patentamt / European Patent Office / Office européen des brevets

(11) **EP 4 154 215 B1**

(12) **FASCICULE DE BREVET EUROPEEN**

(45) Date de publication et mention de la délivrance du brevet:
**01.04.2026 Bulletin 2026/14**

(21) Numéro de dépôt: **21732482.1**

(22) Date de dépôt: **18.05.2021**

(51) Classification Internationale des Brevets (IPC):
**G06T 7/00** *(2017.01)*    **A61B 5/055** *(2006.01)*
**G01R 33/54** *(2006.01)*    **G01R 33/56** *(2006.01)*
**G01R 33/563** *(2006.01)*

(52) Classification Coopérative des Brevets (CPC):
**G06T 7/0012; A61B 5/055; G01R 33/5608;**
G01R 33/546; G01R 33/56341; G06T 2207/10088;
G06T 2207/30081

(86) Numéro de dépôt international:
**PCT/FR2021/050872**

(87) Numéro de publication internationale:
**WO 2021/234278 (25.11.2021 Gazette 2021/47)**

(54) **SYSTÈME ET PROCÉDÉ POUR ESTIMER UN INDICATEUR D'ACTIVITÉ TISSULAIRE D'UN ORGANE**

SYSTEM UND VERFAHREN ZUR SCHÄTZUNG EINES INDIKATORS DER GEWEBEAKTIVITÄT EINES ORGANS

SYSTEM AND METHOD FOR ESTIMATING AN INDICATOR OF THE TISSUE ACTIVITY OF AN ORGAN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.05.2020 FR 2005142**

(43) Date de publication de la demande:
**29.03.2023 Bulletin 2023/13**

(73) Titulaire: **Olea Medical**
**13600 La Ciotat (FR)**

(72) Inventeurs:
• **SUDRE, Romain**
  **34000 MONTPELLIER (FR)**
• **ROUYER, Julien**
  **13100 AIX-EN-PROVENCE (FR)**
• **BOUTELIER, Timothé**
  **13600 CEYRESTE (FR)**
• **AVARE, Christophe**
  **13600 CEYRESTE (FR)**

(74) Mandataire: **Brun, Philippe Alexandre Georges**
**Med'inVent Consulting**
**Espace Mistral - Bât.A**
**297 avenue du Mistral**
**ZI ATHELIA IV**
**13705 La Ciotat Cedex (FR)**

(56) Documents cités:
**EP-A1- 3 387 457**    **EP-B1- 3 387 457**
**WO-A1-2019/063342**    **US-A1- 2019 250 235**

**Description**

L'invention concerne un système et un procédé pour quantifier un nouveau biomarqueur de l'activité tissulaire d'un organe humain ou animal. A titre d'exemple d'application préférée, un tel biomarqueur décrit la diffusivité de fluides biologiques dans des tissus vivants sous la forme d'un nouvel indicateur de la diffusion de molécules d'eau dans des tissus vivants à partir de données de diffusion résultant de l'acquisition d'une séquence d'images d'une ou plusieurs parties du corps d'un patient animal ou humain. Dans le cadre de cet exemple d'application préférée, un tel biomarqueur est nommé ci-après « indicateur d'activité tissulaire » ou TAI, acronyme de l'expression anglo-saxonne « *Tissue Activity Indicator ».*

L'invention s'appuie notamment sur des techniques d'imagerie par Résonance Magnétique (*Magnetic Resonance Imaging* ou MRI, selon une terminologie anglo-saxonne), plus particulièrement l'imagerie pondérée de Diffusion (*Diffusion Weighted Imaging* ou DWI, selon une terminologie anglo-saxonne) ou par Tomodensitométrie (*Computed Tomography* ou CT, selon une terminologie anglo-saxonne). Ces techniques permettent d'obtenir rapidement des informations précieuses sur les mouvements des molécules d'eau au sein d'organes ou de tissus présents chez les êtres humains ou les animaux. Ces informations sont particulièrement cruciales pour un praticien cherchant à établir un diagnostic et à prendre une décision thérapeutique dans le traitement de pathologies.

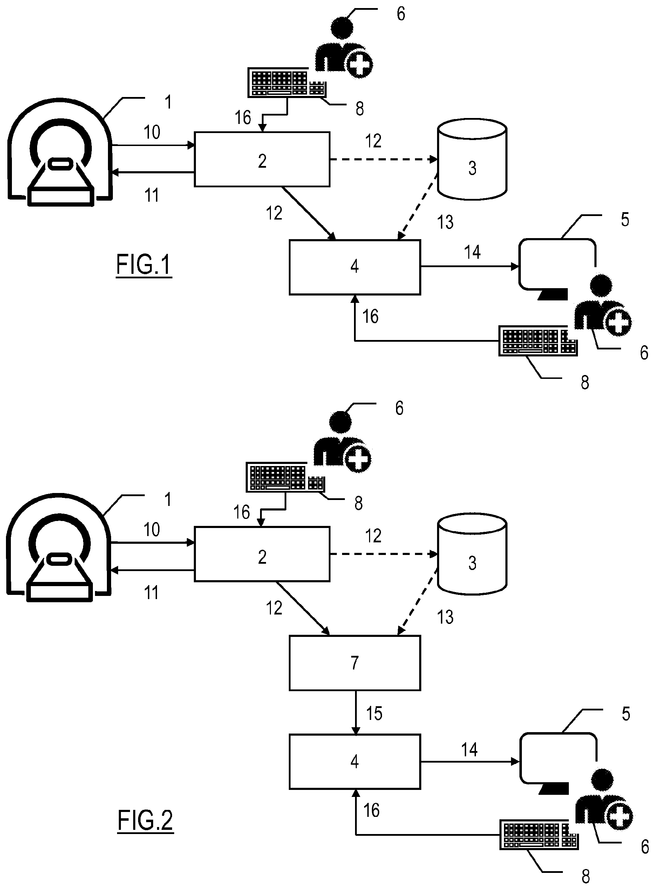

Pour mettre en œuvre de telles techniques, un appareil d'imagerie 1 par Résonance Magnétique Nucléaire, tel qu'illustré à titre d'exemple non limitatif par les figures 1 et 2, est généralement utilisé. Celui-ci peut délivrer une pluralité de séquences d'images numériques 12 d'une ou plusieurs parties du corps d'un patient, à titre d'exemples non limitatifs, le cerveau, le cœur, les poumons. Ledit appareil applique pour cela une combinaison d'ondes électromagnétiques à haute fréquence sur la partie du corps considérée et mesure le signal réémis par certains atomes, tels qu'à titre d'exemple non limitatif, l'hydrogène pour l'imagerie par Résonance Magnétique Nucléaire. L'appareil permet ainsi de déterminer les propriétés magnétiques et, par voie de conséquence, la composition chimique des tissus biologiques et donc leur nature, en chaque volume élémentaire, que l'on nomme communément un voxel, du volume imagé. L'appareil 1 d'imagerie par Résonance Magnétique Nucléaire est commandé à l'aide d'une console 2. Un utilisateur 6, par exemple un opérateur, praticien ou chercheur, peut ainsi choisir des commandes 11 pour piloter l'appareil 1, à partir de paramètres ou de consignes 16 saisis via une interface homme-machine d'entrée 8 du système d'analyse. Une telle interface homme-machine 8 peut consister par exemple en un clavier informatique, un dispositif de pointage, d'un écran tactile, un microphone ou, plus généralement, toute interface agencée pour traduire une gestuelle ou une consigne émise par un humain 6 en données de commande ou de paramétrage. A partir d'informations 10 produites par ledit appareil 1, on obtient une pluralité de séquences d'images numériques 12 d'une partie d'un corps d'un humain ou d'un animal. Nous nommerons également « données expérimentales » de telles informations 10 ou images 12.

Les séquences d'images 12 peuvent optionnellement être stockées au sein d'un serveur 3 et constituer un dossier médical 13 d'un patient. Un tel dossier 13 peut comprendre des images de différents types, telles que des images fonctionnelles mettant en évidence l'activité des tissus, ou des images anatomiques reflétant les propriétés des tissus. Les séquences d'images 12 ou, plus généralement, les données expérimentales sont analysées par une unité de traitement 4 agencée à cette fin, sous la forme par exemple d'un ou plusieurs microprocesseurs ou microcontrôleurs mettant en œuvre des instructions de programmes d'application idoines chargées dans des moyens de mémorisation, tels qu'une mémoire non volatile, dudit système d'analyse d'imagerie. Ladite unité de traitement 4 comporte des moyens pour communiquer avec le monde extérieur pour recueillir les images. Lesdits moyens pour communiquer permettent en outre à l'unité de traitement 4 de délivrer in fine, par l'intermédiaire d'une interface homme-machine de sortie 5 à un utilisateur 6 du système d'analyse d'imagerie, un rendu, par exemple graphique et/ou sonore, d'une estimation ou d'une quantification d'un biomarqueur élaborée par ladite unité de traitement 4 à partir des données expérimentales 10 et/ou 12 obtenues par Imagerie par Résonance Magnétique. Dans tout le document, on entend par « interface homme-machine de sortie », tout dispositif, employé seul ou en combinaison, permettant de sortir ou de délivrer une représentation graphique, haptique, sonore ou, plus généralement, perceptible par l'humain, d'un signal physiologique reconstruit, en l'espèce un biomarqueur, à un utilisateur 6 d'un système d'analyse d'Imagerie par Résonance Magnétique. Une telle interface homme-machine de sortie 5 peut consister de manière non exhaustive en un ou plusieurs écrans, haut-parleurs ou autres moyens alternatifs adaptés. Ledit utilisateur 6 du système d'analyse peut ainsi confirmer ou infirmer un diagnostic, décider d'une action thérapeutique qu'il jugera adéquate, approfondir des travaux de recherche, etc. Optionnellement, cet utilisateur 6 peut paramétrer le fonctionnement de l'unité de traitement 4 ou de l'interface homme-machine de sortie 5, au moyen de paramètres 16 de fonctionnement et/ou d'acquisition. Par exemple, il peut ainsi définir des seuils d'affichage ou choisir les biomarqueurs, indicateurs ou paramètres estimés ou quantifiés pour lesquels il souhaite disposer d'une représentation. L'utilisateur exploite pour cela l'interface homme-machine d'entrée 8 précédemment évoquée ou une deuxième interface d'entrée prévue pour cela. Avantageusement, les interfaces homme-machine d'entrée 8 et de sortie 5 peuvent ne constituer qu'une seule et même entité physique. Lesdites interfaces homme-machine d'entrée 8 et de sortie 5 du système d'analyse d'imagerie peuvent également être intégrées à la console d'acquisition 2. Il existe une variante, décrite en liaison avec la figure 2, pour laquelle un système d'imagerie, tel que décrit précédemment, comporte en outre une unité de prétraitement 7 pour analyser les séquences d'images 12, en déduire des signaux expérimentaux 15 et délivrer ces derniers à l'unité de traitement 4 qui est ainsi déchargée de cette tâche.

L'acquisition d'une ou plusieurs données expérimentales, avantageusement et respectivement un ou plusieurs signaux expérimentaux, par Imagerie de Résonance Magnétique, ci-après dénommée IRM, peut être effectuée en échantillonnant régulièrement un volume parallélépipédique selon un plan de coupe donné, parmi lesquels nous pouvons citer les plans de coupe coronal, sagittal et axial. Les images en deux dimensions obtenues sont constituées de pixels dotés d'une épaisseur correspondant à l'épaisseur de la coupe et appelés voxel. Une telle technique d'imagerie permet ainsi de pouvoir acquérir des images aussi bien anatomiques, pour permettre par exemple de refléter les propriétés des tissus, que fonctionnelles, pour mettre en évidence par exemple l'activité des tissus.

Parmi les techniques ou modalités basées sur l'imagerie par résonance magnétique, on distingue l'imagerie par résonance magnétique de diffusion. L'imagerie de diffusion apporte des informations non disponibles sur les séquences d'imagerie conventionnelle. Elle permet, à titre d'exemple non limitatif, le diagnostic précoce des accidents vasculaires ischémiques et constitue une aide au diagnostic de lésions cancéreuses. Elle apporte également des informations pronostiques, en permettant, par exemple, de distinguer un œdème vasogénique généralement réversible, d'un œdème cytotoxique généralement irréversible. La diffusion observée étant contrainte par les tissus environnants, cette modalité d'imagerie permet notamment d'estimer indirectement la position, l'orientation et l'anisotropie des tissus ou structures fibreuses, telles qu'à titre d'exemples non limitatifs les faisceaux de matière blanche du cerveau. L'imagerie par résonance magnétique étant principalement basée sur l'analyse de la réponse des atomes d'hydrogène des molécules d'eau, c'est principalement la diffusion des molécules d'eau qui est observée par cette modalité sous la forme d'un biomarqueur déterminé, également qualifié de « paramètre d'intérêt ».

Parmi les séquences couramment employées en imagerie de diffusion, on peut distinguer la séquence d'imagerie de diffusion multi-b qui s'attache à donner une ou plusieurs informations sur les mouvements des molécules d'eau présentes dans l'organisme d'un être vivant, généralement un être animal ou humain. Dans un milieu liquide ouvert par exemple, les molécules d'eau sont généralement animées par un mouvement brownien : on parle alors de diffusion libre. Au contraire, dans les tissus vivants, les mouvements de fluide sont contraints par plusieurs facteurs physiques, tels que, par exemple, les variations de pression hydrostatique et de pression osmotique mais aussi par des facteurs structurels tels que, par exemple, la densité cellulaire des tissus et la présence de macromolécules au sein desdits tissus.

L'évaluation de la diffusion en un voxel donné repose sur une acquisition itérative pour différentes valeurs d'un paramètre d'acquisition, en l'espèce l'intensité d'un gradient de champ magnétique, ci-après nommé « le gradient de diffusion », communément qualifié « paramètre b », défini tel que $b = \gamma^2 g_D^2 \delta^2 \left( \Delta - \dfrac{\delta}{3} \right)$ où $\gamma$ consiste en le rapport gyromagnétique et les paramètres suivants définissent les caractéristiques du gradient impulsionnel : l'amplitude $g_D$, la durée de l'impulsion $\delta$ et le temps $\Delta$ entre deux impulsions, également nommé « temps de répétition ».

Généralement, deux gradients de diffusion successifs sont utilisés pour réaliser une mesure à une valeur de b donnée. Initialement, en cohérence de phase, les spins nucléaires des protons d'hydrogène des molécules d'eau sont déphasés par un premier gradient d'impulsions magnétiques de diffusion, alors qu'un second gradient d'impulsions magnétiques de diffusion permet de replacer lesdits spins nucléaires en cohérence de phase dans leur état initial. Une telle réversibilité de l'état initial n'est possible qu'à la condition que le phénomène de diffusion soit nul. Dans les cas où un phénomène de diffusion existe, le mouvement stochastique des molécules d'eau conduit à une incapacité du second gradient à produire un retour des spins à leur état initial.

Ainsi, la distribution aléatoire des phases entraîne une chute de l'intensité du signal acquis puisque ce dernier résulte de la somme des contributions de chacun des spins présents dans le voxel considéré. Une telle perte de l'intensité d'un signal est traitée comme une atténuation. Lorsque la valeur de b augmente, le temps de répétition $\Delta$ augmente par définition : un plus grand déphasage est alors observé, se traduisant par une plus forte atténuation de l'intensité du signal. L'atténuation de l'intensité du signal, communément qualifiée d'atténuation du signal, telle qu'observée peut être modélisée comme le rapport du signal mesuré $S(D,b)$ et du signal obtenu sans gradient de diffusion $S_0$, c'est-à-dire lorsque b est nul. Ce rapport possède un comportement exponentiel fonction d'un biomarqueur spécifique, le coefficient de diffusion D, et du paramètre b, tel que $\dfrac{S(D,b)}{S_0} = e^{-bD}$ .

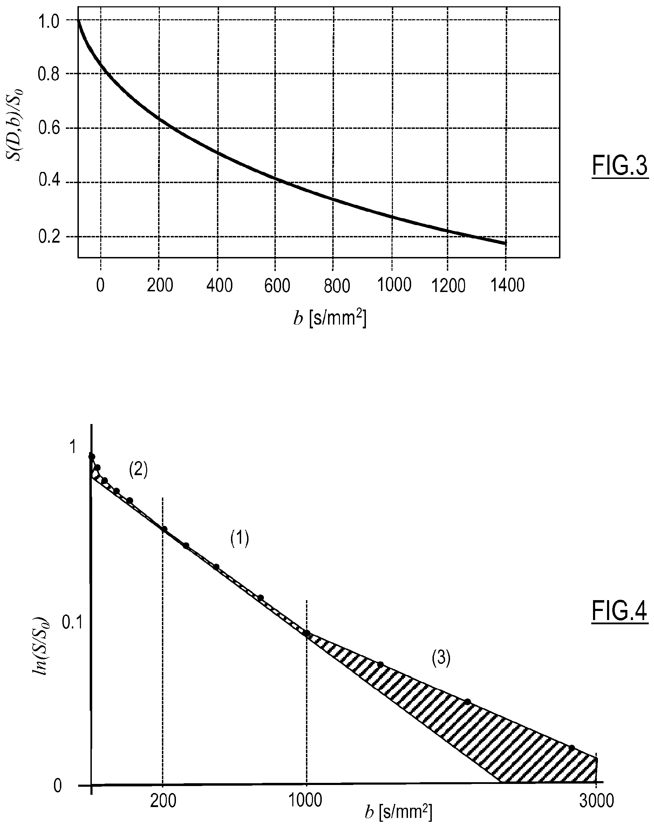

Un tel modèle repose sur l'hypothèse de diffusion gaussienne des molécules d'eau présente dans les tissus biologiques : la dimension du paramètre b correspond ainsi à la dimension inverse du coefficient de diffusion D et s'exprime en $s/mm^2$. La figure 3 illustre un exemple d'une courbe d'atténuation de l'intensité du signal, plus particulièrement la dépendance exponentielle de l'atténuation du signal en fonction du paramètre b pour un coefficient de diffusion D de $1.3 \times 10^{-3}$ $mm^2/s$.

Pour permettre d'exploiter la courbe d'atténuation du signal et, in fine, estimer un ou plusieurs biomarqueurs, de tels biomarqueurs fournissant une information sur le métabolisme des tissus, plusieurs chercheurs ont proposé différentes modélisations analytiques de la diffusion. A titre d'exemples non limitatifs, un tel biomarqueur peut consister en un coefficient de diffusion.

A l'heure actuelle, un des modèles les plus employés dans le domaine clinique consiste en le modèle mono-exponentiel. Un tel modèle mono-exponentiel repose sur l'exploitation de la modélisation du rapport du signal mesuré $S(D,b)$ et du signal obtenu sans gradient de diffusion $S_0$, où le coefficient de diffusion est interprété comme un coefficient de diffusion apparent ADC, défini tel que $\ln\left[\frac{S(D,b)}{S_0}\right] = -bADC$ .

En variante, d'autres praticiens emploient le concept et modèle IVIM (acronyme pour « *IntraVoxel Incoherent Motion* »), également qualifié de modèle bi-exponentiel, initialement introduit et développé par Monsieur Denis LE BIHAN. Un tel modèle permet notamment d'évaluer quantitativement tous les mouvements de translation microscopiques qui pourraient contribuer au signal acquis par l'IRM de diffusion. Dans ce modèle, le tissu biologique contient deux environnements distincts : la diffusion moléculaire de l'eau dans le tissu, également qualifiée de « diffusion réelle », et la microcirculation du sang dans le réseau capillaire, également qualifiée de « perfusion ». Le concept IVIM consiste à considérer que l'eau s'écoulant dans les capillaires, pour un voxel donné, imite une marche aléatoire, qualifiée également de « pseudodiffusion », tant que l'hypothèse selon laquelle toutes les directions sont représentées dans les capillaires, c'est-à-dire qu'il n'y a pas de flux net cohérent dans aucune direction, est satisfaite. Le flux sanguin dans les capillaires, que l'on qualifie de « phénomène de perfusion », imite un processus de diffusion et a un impact sur les mesures acquises par IRM de diffusion : il est ainsi responsable d'une atténuation du signal en IRM de diffusion qui dépend de la vitesse du flux sanguin et de l'architecture vasculaire. L'effet de la pseudodiffusion sur l'atténuation du signal dépend de la valeur b. Cependant, le taux d'atténuation du signal résultant de la pseudodiffusion est généralement d'un ordre de grandeur supérieur à la diffusion moléculaire dans les tissus, de sorte que sa contribution relative au signal IRM pondéré en fonction de la diffusion ne devient significative qu'à des valeurs b très faibles, ce qui permet de séparer les effets de diffusion et de perfusion. En effet, pour des faibles valeurs de b entre 0 et 200 s/mm$^2$, les techniques d'imagerie de diffusion multi-b ne sont pas seulement sensibles aux phénomènes de diffusion mais deviennent également sensibles aux phénomènes de perfusion capillaire. Dans ce cas, l'atténuation du signal peut alors être modélisée par une fonction bi-exponentielle, telle que

$$\frac{S(D, D^*, f, K, b)}{S_0} = f\exp(-bD^*) + (1-f)\exp(-bD)$$

où f consiste en la fraction de perfusion, D consiste en le coefficient de diffusion et D* consiste en le coefficient de pseudodiffusion.

En variante ou en complément, un troisième modèle a été employé pour prendre en compte le comportement non gaussien de la diffusion dans un tissu : un tel modèle est qualifié de modèle de Kurtosis. En effet, pour de très grandes valeurs de b allant au-delà de 1000 s/mm$^2$, le régime de diffusion gaussien s'interrompt. Alors que l'hypothèse de diffusion gaussienne est correcte dans les liquides et/ou les gels dont les mouvements sont libres, une telle hypothèse devient erronée dès lors que le fluide est contraint par des barrières limitant le déplacement libre des molécules. Cette diffusion contrainte, également qualifiée de diffusion restreinte, est notamment observée dans les tissus biologiques puisque la membrane des cellules compartimente le fluide interstitiel. Ainsi, aux grandes valeurs de b, c'est-à-dire après un certain temps laissé pour la réalisation du phénomène de diffusion, l'atténuation du signal dévie du modèle gaussien. Des chercheurs ont ainsi proposé une généralisation du modèle IVIM de sorte à prendre en compte l'effet de déviation, telle que $\frac{S(D,D^*,f,K,b)}{S_0} = f\exp(-bD^*) + (1-f)\exp\left(-bD + b^2D^2\frac{K}{6}\right)$ où K consiste en le coefficient adimensionnel de Kurtosis caractérisant le degré de déviation au modèle gaussien.

La figure 4 illustre un exemple de représentation logarithmique de l'atténuation du signal, c'est-à-dire le rapport du signal mesuré $S(D,b)$ et du signal obtenu sans gradient de diffusion $S_0$, sur une large gamme de valeur de b, allant de 0 à 3000 s/mm$^2$. Une telle représentation se décompose principalement en trois phases, référencées (1), (2) et (3) sur la figure 4, et permet notamment de visualiser les deux déviations, correspondant aux plages de valeurs de b associées aux phases (2) et (3) sur la figure 4, au modèle gaussien ou modèle mono-exponentiel, correspondant à la plage de valeurs de b associée à la phase (1) sur la figure 4. De telles déviations sont notamment observées respectivement, dans les petites valeurs de b (correspondant à la phase (2) sur la figure 4) de l'ordre 0 à 200 s/mm$^2$, la déviation étant alors causée par l'effet de perfusion, et dans les grandes valeurs de b (correspondant à la phase (3) sur la figure 4), de l'ordre 1000 à 3000 s/mm$^2$, la déviation étant alors causée par l'effet de diffusion restreinte.

L'emploi des modèles analytiques précédemment décrits en vue d'exploiter les courbes d'atténuation permet notamment de produire une description intéressante du phénomène de diffusion dans les tissus à différentes échelles biologiques. Cependant, la mise en œuvre de tels modèles analytiques n'est généralement pas aisée et la précision des estimations est fortement liée à la qualité des données ou des signaux acquis. Généralement, trois facteurs principaux handicapent leur usage :

- un temps de calcul important qui peut s'avérer rédhibitoire en usage clinique ;
- un niveau élevé du bruit dans les données ou signaux acquis et issus des séquences d'acquisition de diffusion multi-b ;
- le faible nombre de valeurs de b fournis par les séquences d'acquisition de diffusion multi-b actuelles.

Il existe de nombreuses techniques d'estimation d'un biomarqueur sous la forme d'un coefficient de diffusion apparent ADC, comme le divulgue à titre d'exemple le document EP 3387457 A1. De telles techniques sont très largement utilisée à l'heure actuelle dans le domaine clinique. Une telle estimation par un modèle mono-exponentiel, repose principalement sur des signaux et/ou données expérimentales provenant de l'acquisition de deux valeurs de b principales : b = 0 s/mm$^2$, c'est-à-dire sans gradient de diffusion, et b = 1000 s/mm$^2$ pondérée en diffusion. Elle requiert un faible échantillonnage et en conséquence des temps d'acquisition relativement courts. Toutefois, une telle estimation du coefficient de diffusion apparent ADC s'avère fortement dégradée dès lors que le niveau de bruit devient important. De plus, un tel biomarqueur est généralement peu sensible aux différents phénomènes qui peuvent affecter le signal de diffusion comme, par exemple, les effets IVIM ou Kurtosis mentionnés précédemment, et, en conséquence, peu représentatif desdits phénomènes.

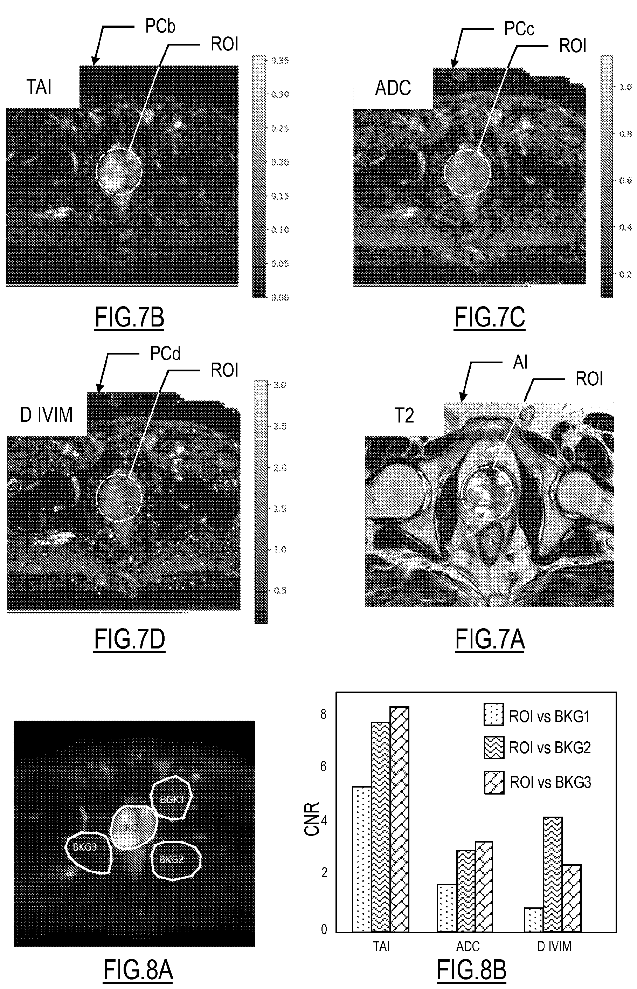

L'obtention d'images ou de cartes paramétriques représentatives d'un biomarqueur estimé en lien avec un modèle IVIM ou un modèle de Kurtosis permet notamment de montrer les différents régimes de diffusion et ainsi, d'informer un praticien ou, plus largement un opérateur, des différents processus microscopiques qui peuvent avoir alors lieu au sein d'un tissu animal ou humain, pour, *in fine,* permettre d'établir un diagnostic et prendre une décision thérapeutique dans le traitement de pathologies. Néanmoins, les temps de calculs relatifs à des estimations d'un ou plusieurs biomarqueurs en lien avec un modèle IVIM ou un modèle de Kurtosis s'avèrent relativement longs et les cartes paramétriques obtenues représentatives de tels biomarqueurs sont généralement très sensibles au bruit, en particulier les cartes représentatives de la fraction de perfusion f, du coefficient de pseudodiffusion D* et du coefficient adimensionnel de Kurtosis K, comme nous l'étudierons plus loin en lien avec les figures 7C, 7D et 8B. Par ailleurs, le faible échantillonnage des courbes d'atténuation du signal n'offre pas des conditions favorables à l'emploi des modèle IVIM ou modèle de Kurtosis : en effet, les régressions employées en lien avec un faible échantillonnage peuvent conduire à des estimations biaisées, diminuant leur fiabilité et leur robustesse.

A l'heure actuelle, il n'existe pas de procédé pour estimer un biomarqueur à partir d'une donnée expérimentale, notamment de diffusion, permettant de rendre compte de manière rapide, robuste et fiable de phénomènes microscopiques, plus particulièrement des mouvements des molécules d'eau, mis en évidence au sein d'un organe ou tissu animal ou humain par les séquences d'imagerie, notamment de diffusion.

L'invention permet de répondre à tout ou partie des inconvénients soulevés par les solutions connues ou précédemment mentionnées.

Parmi les nombreux avantages apportés par l'invention, nous pouvons mentionner que celle-ci permet de :

- quantifier un nouveau biomarqueur de l'activité tissulaire particulièrement résistant et stable aux bruits présents dans les signaux d'imagerie médicale dont découlent les données expérimentales ;
- procurer un nouveau biomarqueur exploitable et pertinent dans un grand nombre d'applications parmi lesquelles, nous pouvons citer, de manière non exhaustive, l'analyse et/ou le suivi de cancers, l'évaluation d'accidents vasculaires cérébraux ;
- quantifier un biomarqueur à partir de données expérimentales brutes, du fait de la très faible sensibilité du procédé de quantification au bruit, au paramétrage d'acquisition et à la disparité des systèmes d'imagerie ;
- quantifier un biomarqueur ne nécessitant pas d'importantes ressources de calcul ou un temps de calcul prohibitif, contrairement aux biomarqueurs connus du fait de l'exploitation de modèles théoriques pour estimer ces derniers ;
- adapter ou paramétrer la quantification d'un nouveau biomarqueur selon les attentes des patriciens, les modalités d'acquisition, les organes examinés.

Selon un premier objet, l'invention prévoit un procédé pour quantifier un biomarqueur d'un volume élémentaire, dit « voxel » d'un organe, ledit procédé étant mis en œuvre par une unité de traitement d'un système d'analyse d'imagerie d'IRM de diffusion, et comportant une étape pour produire la valeur dudit biomarqueur, ci-après désigné « indicateur d'activité tissulaire » ou TAI, à partir de données expérimentales S(b).

Pour résoudre les inconvénients précédemment évoqués en lien avec les techniques connues, l'étape pour produire la valeur du biomarqueur TAI consiste, sur un intervalle borné $b_{min}$ à $b_{max}$, de valeurs d'un paramètre d'acquisition b correspondant à l'intensité du gradient de diffusion, en le calcul $TAI = \int_{b_{min}}^{b_{max}} L(b) - \Gamma_S[S(b)]\, db$, L(b) étant une fonction dudit paramètre d'acquisition b et $\Gamma_S[S(b)]$ une transformation bijective desdites données expérimentales S(b).

Selon un mode de réalisation avantageux, lesdites fonction et transformation bijective peuvent être mutuellement déterminées de sorte que L(b) soit supérieure ou égale à $\Gamma_S[S(b)]$, sur l'ensemble des valeurs du paramètre d'acquisition b comprises entre $b_{min}$ et $b_{max}$.

En variante ou en complément, lesdites fonction et transformation bijective peuvent être mutuellement déterminées, de sorte que $L(b_{min})=\Gamma_S[S(b_{min})]$ et/ou $L(b_{max}) = \Gamma_S[S(b_{max})]$.

Pour faciliter l'exploitation du biomarqueur TAI par tout utilisateur d'un système d'analyse d'imagerie ainsi adapté, lorsque ce dernier comporte une interface homme-machine de sortie, un procédé conforme à l'invention peut comporter une étape subséquente pour déclencher une sortie dudit biomarqueur quantifié selon un format approprié.

Pour adapter ou paramétrer la quantification du biomarqueur selon les attentes des patriciens ou opérateurs, les modalités d'acquisition, les organes examinés, lorsque le système d'analyse d'imagerie comporte une interface homme-machine d'entrée, un procédé conforme à l'invention peut comporter une étape de détermination de la fonction L(b) dudit paramètre d'acquisition b et de la transformation bijective $\Gamma_S[S(b)]$ desdites données expérimentales S(b) à partir de données d'entrée d'un utilisateur de ladite interface homme-machine d'entrée.

Pour quantifier un tel biomarqueur pour une pluralité de voxels considérés, l'étape pour produire la valeur dudit biomarqueur peut être mise en œuvre par itérations successives pour une pluralité de voxels considérés, ledit indicateur d'activité tissulaire étant quantifié par voxel.

A titre d'exemple préféré de sortie du biomarqueur, l'étape pour déclencher une sortie de ce dernier peut consister à générer une image sous la forme d'une carte paramétrique dont les pixels encodent respectivement les valeurs dudit biomarqueur quantifié pour les voxels considérés.

Selon un deuxième objet, l'invention prévoit un système d'analyse d'imagerie comportant une unité de traitement, des moyens pour communiquer avec le monde extérieur et des moyens de mémorisation, dont :

- les moyens pour communiquer sont agencés pour recevoir du monde extérieur des données expérimentales S(b) d'un volume élémentaire d'un organe ;
- les moyens de mémorisation comportent des instructions dont l'interprétation ou l'exécution par ladite unité de traitement provoque la mise en œuvre d'un procédé pour quantifier un biomarqueur d'un volume élémentaire dudit organe conforme à l'invention et évoqué ci-avant.

A titre d'exemple d'application préférée :

- les données expérimentales S(b) d'un volume élémentaire d'un organe peuvent être des données résultant d'une acquisition d'un signal par imagerie de diffusion ;
- le biomarqueur quantifié peut être un indicateur de la diffusion de molécules d'eau dans un volume élémentaire dudit organe.

Selon un troisième objet, l'invention prévoit un produit programme d'ordinateur comportant une ou plusieurs instructions interprétables ou exécutables par l'unité de traitement d'un système d'analyse d'imagerie conforme à l'invention, ledit programme étant chargeable dans des moyens de mémorisation dudit système, caractérisé en ce que l'interprétation ou l'exécution desdites instructions par ladite unité de traitement provoque la mise en œuvre d'un procédé pour quantifier un biomarqueur d'un volume élémentaire selon l'invention.

D'autres caractéristiques et avantages apparaîtront plus clairement à la lecture de la description qui suit et à l'examen des figures qui l'accompagnent parmi lesquelles :

- la figure 1, d'ores et déjà décrite, illustre une description simplifiée d'un système d'analyse d'images obtenues par Résonance Magnétique Nucléaire ;
- la figure 2, d'ores et déjà décrite, illustre une description simplifiée d'une variante d'un système d'analyse d'images obtenues par Résonance Magnétique Nucléaire ;
- la figure 3, d'ores et déjà décrite, illustre un exemple d'une courbe d'atténuation de l'intensité d'une donnée expérimentale en fonction d'un paramètre d'acquisition ;
- la figure 4, d'ores et déjà décrite, illustre un exemple de représentation logarithmique de l'atténuation de l'intensité d'une donnée expérimentale en fonction d'un paramètre d'acquisition ;
- la figure 5 illustre une description simplifiée d'un procédé conforme à l'invention pour quantifier un biomarqueur d'un organe ;
- les figures 6A, 6B et 6C illustrent des variantes d'une quantification d'un biomarqueur conforme à l'invention à partir de données expérimentales d'un voxel d'un organe ;
- la figure 7A illustre une image anatomique comportant une région d'intérêt, en l'espèce une prostate, et obtenue par une séquence T2 ;

- la figure 7B illustre, sous la forme d'une image, le rendu graphique d'un biomarqueur quantifié pour chaque voxel de la prostate évoquée précédemment en lien avec la figure 7A, à partir de données expérimentales de diffusion, selon un procédé conforme à l'invention ;
- la figure 7C illustre, sous la forme d'une image, le rendu graphique d'un biomarqueur estimé pour chaque voxel de la prostate évoquée précédemment en lien avec la figure 7A, à partir de données expérimentales de diffusion et un modèle mono-exponentiel selon un procédé conforme à l'art antérieur ;
- la figure 7D illustre, sous la forme d'une image, le rendu graphique d'un biomarqueur estimé pour chaque voxel de la prostate évoquée précédemment en lien avec la figure 7A, à partir de données expérimentales de diffusion et un modèle bi-exponentiel de type IVIM (acronyme anglo-saxon pour « *IntraVoxel Incoherent Motion* ») selon un procédé conforme à l'art antérieur ;
- la figure 8A illustre sous la forme d'une image, la détermination de quatre régions à partir d'une représentation graphique telle que celle décrite en lien avec la figure 7B, obtenue par la quantification d'un biomarqueur conforme à l'invention, lesdites quatre régions décrivant une région d'intérêt focalisée sur la prostate et trois autres régions adjacentes à la précédente ;
- la figure 8B illustre, sous la forme d'histogrammes, les ratios « contraste sur bruit » lors de la discrimination de ladite région d'intérêt au regard de chacune des trois autres régions évoquées précédemment, lorsque l'image source est celle obtenue par la quantification d'un biomarqueur (cf. figure 7B) selon l'invention ou selon l'art antérieur, à partir d'un modèle mono-exponentiel (cf. figure 7C) ou bi-exponentiel (cf. figure 7D).

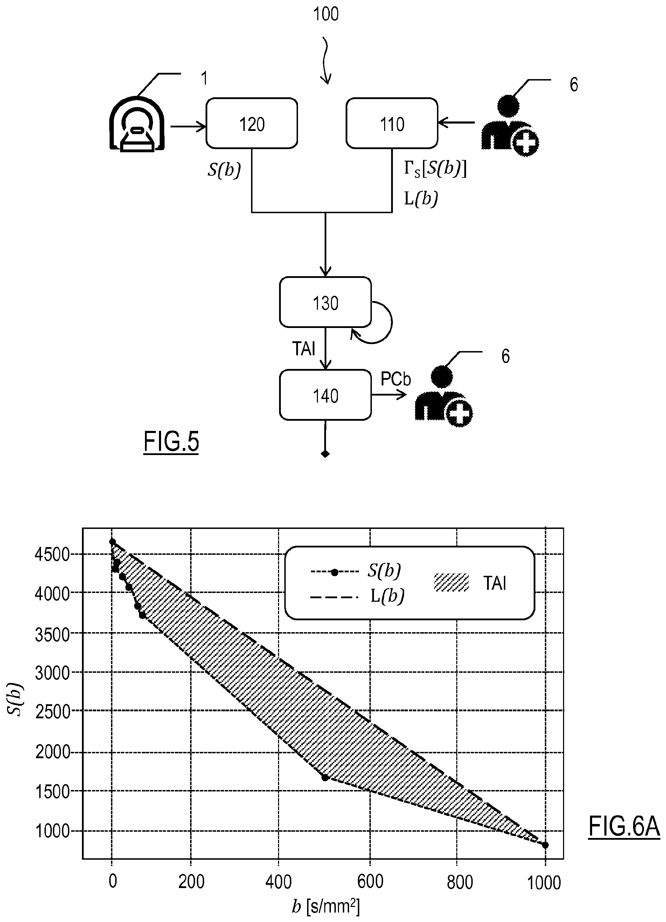

Décrivons à présent, en lien avec les figures 5 et 6A, un exemple de réalisation préférée mais non limitatif d'un procédé, conforme à l'invention, pour quantifier un nouveau biomarqueur TAI, ledit biomarqueur s'apparentant à un indicateur d'activité tissulaire, obtenu à partir de données expérimentales de diffusion d'un organe, par exemple une prostate chez l'homme. L'invention ne saurait être limitée à ces seuls exemples de modalités d'acquisition, d'organe et pourrait s'appliquer à l'animal.

Un tel procédé 100 est destiné à être mis en œuvre par une unité de traitement d'un système d'analyse d'imagerie médicale tel que celui illustré précédemment en lien avec la figure 1 ou la figure 2.

Il comporte principalement une étape 130 pour produire un biomarqueur TAI à partir de données expérimentales S(b) pour chaque voxel d'intérêt d'un organe. De telles données expérimentales peuvent être produites en une étape préalable 120 à partir d'une acquisition de signaux par imagerie d'IRM de diffusion, selon un paramètre d'acquisition b, en l'espèce l'intensité du gradient de diffusion, communément qualifié « paramètre b ». Une telle étape 130 peut être mise en œuvre de manière itérative pour quantifier un tel biomarqueur TAI pour un ensemble de voxels d'intérêt.

Un procédé 100 conforme à l'invention comporte en outre, à l'instar de procédés d'estimation d'autres biomarqueurs issus de l'art antérieur, une étape 140 pour encoder la valeur ou les valeurs du biomarqueur TAI quantifié pour un ou une pluralité de voxels sous la forme d'un contenu graphique, par exemple sous la forme d'une carte paramétrique. Une telle carte paramétrique peut se présenter sous la forme d'un tableau de pixels, ou communément nommé « image », à l'instar de l'exemple de la carte paramétrique PCb illustrée par la figure 7B. Chaque pixel de ladite carte paramétrique PCb encode avantageusement un triplet de valeurs entières comprises entre zéro et deux cents cinquante-cinq selon le codage de couleurs RVB, acronyme pour « Rouge Vert Bleu », également connu sous le sigle RGB, acronyme anglo-saxon de « Red Green Blue ». Un tel codage informatique de couleurs est le plus proche des matériels actuellement disponibles. En général, les écrans d'ordinateurs reconstituent une couleur par synthèse additive à partir de trois couleurs primaires, un rouge, un vert et un bleu, formant sur l'écran une mosaïque généralement trop petite pour être discriminée par un œil humain. Le codage RVB indique une valeur pour chacune de ces couleurs primaires. Une telle valeur est généralement codée sur un octet et appartient donc à un intervalle de valeurs entières comprises entre zéro et deux cents cinquante-cinq. L'étape 140 peut consister ainsi à avantageusement encoder la valeur du biomarqueur TAI quantifié pour un voxel d'intérêt sur la base d'un gradient de couleurs, par exemple du bleu au jaune pour encoder ledit biomarqueur de la plus basse valeur vers la plus haute. De cette manière, lorsque l'on s'intéresse à une pluralité de voxels d'un organe, chaque pixel de la carte paramétrique PCb est associé au voxel correspondant afin d'illustrer graphiquement, en deux dimensions, les valeurs respectives du biomarqueur quantifié, pour ladite pluralité de voxels. Les pixels encodant du jaune décrivent les voxels pour lesquels le biomarqueur TAI témoigne d'une haute activité tissulaire, contrairement à ceux encodant du vert témoignant d'une activité tissulaire moyenne ou encore ceux encodant du bleu, témoignant d'une très faible activité tissulaire. Tout autre encodage graphique pourrait être mis en œuvre à l'étape 140, en complément ou en variante, du gradient précédemment évoqué.

Lorsque le système d'analyse d'imagerie mettant en œuvre le procédé 100 comporte une interface homme-machine de sortie, telle qu'un écran d'ordinateur 5 comme illustré par l'exemple de la figure 1 ou 2, l'étape 140 consiste en outre à déclencher une sortie sous la forme avantageuse d'un affichage ou de tout mode de restitution intelligible par l'humain, dudit indicateur d'activité tissulaire quantifié TAI selon une carte paramétrique PCb, comme nous l'avons évoqué précédemment ou selon tout autre format approprié. De cette manière, un utilisateur 6 dudit système d'analyse peut consulter les résultats de ladite quantification du biomarqueur et bénéficier d'une aide à la décision pour un geste thérapeutique, pour établir un diagnostic ou encore pour confirmer ou infirmer un essai clinique.

Pour décrire un exemple de mise en œuvre de l'étape 130 de quantification d'un biomarqueur TAI conforme à l'invention, considérons que les données expérimentales S(b) résultent d'une étape 120 dudit procédé 100, pour produire lesdites données expérimentales à partir d'une acquisition d'un signal par imagerie de diffusion dont le paramètre d'acquisition b est l'intensité du gradient de diffusion.

L'étape 130 consiste alors en le calcul

$$TAI = \int_{b_{min}}^{b_{max}} L(b) - \Gamma_S[S(b)] \, db$$

où L(b) étant une fonction dudit paramètre d'acquisition b et $\Gamma_S[S(b)]$ une transformation bijective desdites données expérimentales S(b). Selon cet exemple, lesdites fonction et transformation bijective peuvent être mutuellement déterminées, de sorte que L(b) soit supérieure ou égale à $\Gamma_S[S(b)]$ sur l'ensemble des valeurs du paramètre d'acquisition b comprises entre $b_{min}$ et $b_{max}$. De cette façon, la valeur du biomarqueur TAI demeure positive entre $b_{min}$ et $b_{max}$. L'invention ne saurait être limitée par ce choix avantageux.

Selon un premier mode de réalisation, la transformation bijective $\Gamma_S[S(b)]$ est la fonction identité. Nous pouvons alors écrire : $\Gamma_S[S(b)] = S(b)$.

La fonction L(b) peut alors être choisie comme la droite qui relie $S(b_{min})$ et $S(b_{max})$, déterminée selon la relation suivante :

$$L(b) = \frac{S(b_{max}) - S(b_{min})}{b_{max} - b_{min}} \cdot b + \frac{b_{max} \cdot S(b_{min}) - b_{min} \cdot S(b_{max})}{b_{max} - b_{min}}$$

Un premier mode de réalisation d'une quantification du biomarqueur TAI, pour un voxel d'intérêt, est illustré par la figure 6A. Le paramètre d'acquisition, en l'espèce l'intensité du gradient de diffusion b, est compris entre les valeurs $b_{min}$ et $b_{max}$, respectivement égales à 0 et 1000 s/mm$^2$.

Les données expérimentales S(b) sont symbolisées par des points sur une ligne en pointillés. La fonction affine L(b) décrit une droite tracée sur la figure 6A sous la forme d'une ligne discontinue. La quantification du biomarqueur TAI correspond en la soustraction, ou la différence, entre l'aire sous la courbe de la fonction L(b) et l'aire sous la courbe des données expérimentales S(b). L'aire résultant de cette soustraction est représentée hachurée sur la figure 6A et correspond à la valeur quantifiée du biomarqueur TAI sous la forme d'un indicateur d'activité tissulaire. Nous pouvons constater qu'avantageusement la fonction affine L(b) a été choisie telle que $L(b_{min})=\Gamma_S[S(b_{min})]=S(b_{min})$ et $L(b_{max})=\Gamma_S[S(b_{max})]=S(b_{max})$.

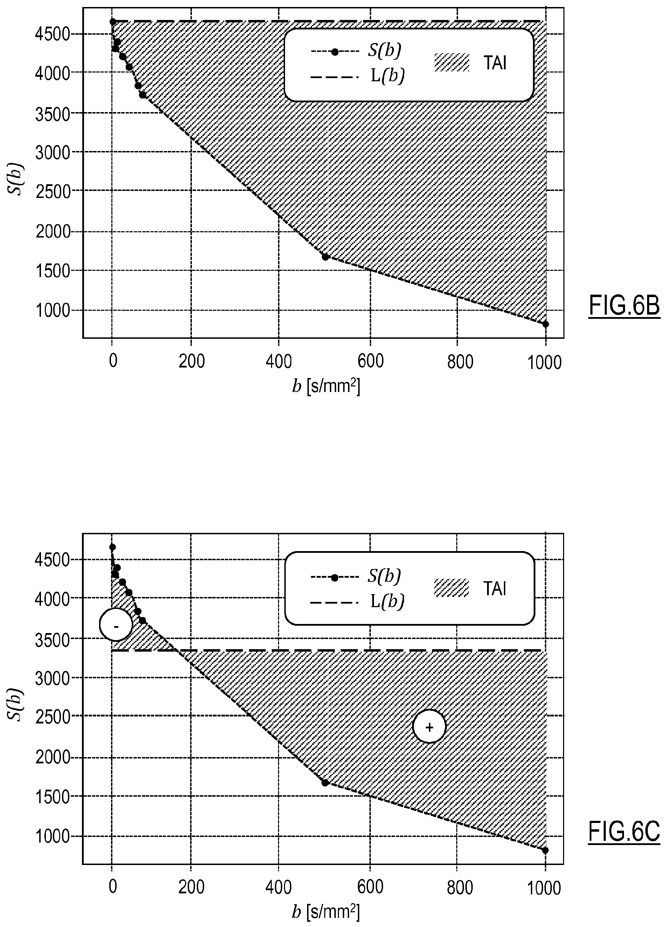

Les figures 6B et 6C illustrent deux variantes d'une quantification du biomarqueur TAI. Selon celles-ci, les données expérimentales S(b) sont identiques à celles exploitées dans le cadre de la quantification selon la figure 6A. En revanche, la fonction L(b) est choisie, c'est-à-dire préétablie, calculée ou paramétrée, telle que celle-ci décrive une constante sur l'intervalle de valeurs $b_{min}$ à $b_{max}$. Ainsi, ladite fonction L(b) selon la figure 6B est telle que L(b) est constante et est égale à une valeur prédéterminée ou déduite des données expérimentales S(b). En l'espèce selon la figure 6B, $L(b) = \max_b S(b)$ sur l'intervalle de valeurs $b_{min}$ à $b_{max}$, c'est-à-dire que L(b) prend la valeur maximale des données expérimentales S(b) sur ledit intervalle des valeurs de b. A l'instar de la figure 6A, la quantification du biomarqueur TAI correspond en la soustraction, ou la différence, entre l'aire sous la courbe de la fonction L(b) et l'aire sous la courbe des données expérimentales S(b). L'aire résultant de cette soustraction est représentée hachurée sur la figure 6B et correspond à la valeur quantifiée du biomarqueur TAI sous la forme d'un indicateur d'activité tissulaire. Une telle constante peut être déterminée ou prédéterminée de manière distincte. Ainsi, comme l'indique la figure 6C, la fonction L(b) peut être choisie telle que $L(b) = \frac{1}{N_b} \cdot \sum_b S(b)$ sur l'intervalle de valeurs de valeurs $b_{min}$ à $b_{max}$, où $N_b$ décrit le nombre d'échantillons ou de données expérimentales considérées. Selon cet exemple illustré par la figure 6C, la quantification du biomarqueur TAI peut se faire de manière signée, en effet, les courbes déterminées par la fonction L(b) et les données expérimentales S(b) sont sécantes autour d'une valeur de b proche de 200s/mm$^2$. La différence entre les aires sous chaque courbes L(b) et S(b) peut être tantôt négative, tantôt positive, comme illustré par la figure 6C pour laquelle des symboles « + » et « - » illustrent ces situations.

Selon une autre technique, une telle quantification du biomarqueur TAI peut être mise en œuvre en choisissant une fonction L(b) polynomiale d'un ordre supérieur ou égal à deux telle que L(b) = $\alpha b^2 + \beta b + \gamma$ pour laquelle :

$$\alpha = -\frac{S(b_{max}) - S(b_{min})}{(b_{max} - b_{min})^2}$$

$$\beta = \frac{S(b_{max}) - S(b_{min})}{b_{max} - b_{min}} - \alpha(b_{max} + b_{min})$$

$$\gamma = S(b_{min}) - \alpha b_{min}^2 - \beta b_{min}$$

En variante, la transformation bijective $\Gamma s[S(b)]$ peut être de nature logarithmique. L'étape 130 du procédé 100 peut produire la valeur du biomarqueur TAI à partir de la transformation $\Gamma_S[S(b)]$ telle que $\Gamma_S[S(b)] = ln\left[\frac{S(b)}{S(b_{min})}\right]$. Selon cette variante, la fonction L(b) peut être définie comme une droite décroissante passant par les deux points $\Gamma_S$ $[S(b_{min})]$ et $\Gamma_S[S(b_{max})]$. L'invention ne saurait être limitée par de tels choix ou paramétrages des fonction et transformation bijective. Toute autre combinaison adaptée à la nature des données expérimentales S(b) pourraient en variante être exploitée, à partir du moment où L(b) demeure une fonction d'un paramètre d'acquisition b et $\Gamma_S[S(b)]$ une transformation bijective desdites données expérimentales S(b).

Pour paramétrer la mise en œuvre de l'étape 130, l'invention prévoit qu'un procédé 100 puisse comporter une étape 110 pour déterminer conjointement la fonction L(b) et la transformation bijective $\Gamma_S[S(b)]$. Lorsque le système d'analyse d'imagerie comporte une interface homme-machine d'entrée, telle que l'interface 8 décrite en lien avec la figure 1 ou la figure 2, ladite interface homme-machine d'entrée coopérant avec l'unité de traitement mettant en œuvre ledit procédé 100, un opérateur 6 peut traduire une gestuelle ou plus généralement une consigne, via ladite interface homme-machine d'entrée 8, pour choisir, par exemple, parmi une banque prédéterminée, saisir et/ou adapter lesdites fonction L(b) et transformation bijective $\Gamma_S[S(b)]$ selon ses us et coutumes, son matériel, l'organe examiné, etc. La détermination conjointe de la fonction L(b) dudit paramètre d'acquisition b et de la transformation bijective $\Gamma s[S(b)]$ desdites données expérimentales S(b) se fait ainsi à partir de données d'entrée dudit utilisateur 6 de ladite interface homme-machine d'entrée 8. L'opérateur 6 peut ainsi aisément optimiser la mise en œuvre du procédé 100 et, par voie de conséquence, la quantification et/ou la sortie du biomarqueur TAI indiquant l'activité tissulaire de l'organe examiné.

Les figures 7A à 7D permettent de mettre en lumière le gain conféré par l'invention au regard des méthodes connues au travers d'un exemple d'application préférée. La figure 7A décrit une image anatomique AI, haute résolution, obtenue par une séquence T2 et comportant une région d'intérêt référencée ROI, en l'espèce une prostate. Ladite région d'intérêt ROI est représentée par un cercle en trait blanc discontinu.

La figure 7B illustre, sous la forme d'une carte paramétrique PCb, le rendu graphique découlant d'une quantification d'un biomarqueur TAI, conforme à l'invention, en lien avec une pluralité de voxels d'intérêt. Pour chaque voxel d'intérêt, ledit biomarqueur TAI a été quantifié par la mise en œuvre d'un procédé, tel que le procédé 100 précédemment décrit, à partir de données expérimentales de diffusion S(b) découlant d'une acquisition d'un signal par imagerie de diffusion. L'image PCb obtenue traduit ledit biomarqueur TAI selon un gradient de couleur allant du bleu sombre au jaune, selon que le biomarqueur comporte une faible ou haute valeur. Nous pouvons constater que la région d'intérêt ROI, la prostate - représentée par un cercle blanc discontinu sur la figure 7B - est clairement discriminée au regard du reste du corps du patient. Le biomarqueur TAI traduit des pixels décrivant principalement une haute activité tissulaire.

La figure 7C illustre, sous la forme d'une carte paramétrique PCc, un biomarqueur, en l'espèce un coefficient de diffusion apparent ADC, estimé pour chacun des mêmes voxels d'intérêt que pour la figure 7B, à partir de données expérimentales de diffusion S(b) similaires mais d'un modèle mono-exponentiel selon un procédé conforme à l'art antérieur. Un même gradient de couleurs (du bleu au jaune) appliqué aux valeurs du biomarqueur ADC permet plus difficilement de discriminer la région d'intérêt ROI, en l'espèce la prostate, du reste du corps. Au sein de ladite région d'intérêt ROI, l'information graphique liée au biomarqueur ADC est également plus diffuse que dans le cas du biomarqueur TAI.

La figure 7D illustre, sous la forme d'une carte paramétrique PCd, un biomarqueur, en l'espèce un coefficient de pseudodiffusion D IVIM, estimé pour chacun des mêmes voxels d'intérêt que pour la figure 7B, à partir de données expérimentales de diffusion S(b) similaires mais d'un modèle bi-exponentiel de type IVIM, acronyme anglo-saxon de « *IntraVoxel Incoherent Motion* » selon un procédé conforme à l'art antérieur. A l'instar de la figure 7C, un même gradient de couleurs (du bleu au jaune) appliqué aux valeurs du biomarqueur D IVIM permet plus difficilement de discriminer la région d'intérêt ROI, en l'espèce la prostate, du reste du corps. Au sein de ladite région d'intérêt ROI, l'information graphique liée au biomarqueur D IVIM est également plus diffuse que dans le cas du biomarqueur TAI et très sensible au bruit, comme en témoigne la présence de nombreux artéfacts de hautes valeurs.

Les figures 8A et 8B permettent d'autant mieux de mesurer le gain conféré par l'invention au regard des techniques connues. En effet, lesdites figures 8A et 8B illustrent une comparaison des performances sur la base d'un critère de contraste sur bruit, également connu par l'acronyme anglo-saxon CNR de « *Contrast-to-Noise Ratio* ». En effet, comme illustré par les figures 7B, 7C et 7D, les biomarqueurs TAI (indicateur d'activité tissulaire) conforme à l'invention, ADC (coefficient de diffusion apparent) du modèle mono-exponentiel ou D IVIM (coefficient de pseudodiffusion) du modèle bi-exponentiel ont été estimés à partir de données expérimentales S(b) tirées de la séquence d'imagerie de diffusion multi-b sans procédure de réduction de bruit, le paramètre d'acquisition b étant compris entre 0 et 1000 s/mm2. Il est courant de comparer des performances sur la base du critère CNR, critère prévalent en imagerie clinique, permettant d'évaluer la détectabilité d'une région d'intérêt par rapport à des régions adjacentes à cette dernière. Un tel critère CNR s'évalue ainsi selon la relation suivante : $CNR = \frac{|\mu_{ROI} - \mu_{BKG}|}{\sigma_{BKG}}$ où $\mu_{ROI}$ et $\mu_{BKG}$ décrivent respectivement des valeurs moyennes de la région d'intérêt ROI et d'une région BKG adjacente de la précédente, $\sigma_{BKG}$ étant la valeur de l'écart-type dans ladite région adjacente BKG.

La figure 8A est une vue partielle d'une carte paramétrique PCb décrivant le biomarqueur TAI et précédemment évoquée en liaison avec la figure 7B permettant de discriminer une prostate du reste du corps d'un patient. Sur la figure 8A, une région d'intérêt ROI est artificiellement ceinturée, pour assurer la compréhension du propos, par une ligne blanche. Elle se focalise sur la prostate de manière plus précise que la région d'intérêt ROI délimitée précédemment par un cercle en trait discontinu sur les figures 7A à 7D. Trois autres régions adjacentes, respectivement référencées BKG1, BKG2 et BKG3, de ladite région d'intérêt ROI ont été ceinturées également par une ligne blanche sur ladite figure 8A.

Pour comparer les performances respectives des biomarqueurs TAI, ADC et D IVIM, un rapport contraste-sur-bruit CNR a été calculé, pour chaque biomarqueur, entre une même région d'intérêt ROI et différentes régions adjacentes BKG1, BKG2 et BKG3, identiques pour les trois biomarqueurs. Pour réaliser cette comparaison de performances, un tel CNR a été calculé à partir de cartes paramétriques PCb, comme l'indique la figure 8A, mais aussi de cartes paramétriques PCc et PCd d'ores et déjà décrites en lien avec les figures 7B à 7D. Ainsi, pour chaque biomarqueur, trois CNR ont été calculés : ROI vs BKG1, ROI vs BKG2 et ROI vs BKG3.

La figure 8B illustre les CNR calculés pour les trois biomarqueurs TAI, ADC et D IVIM. Il est manifeste que la quantification du biomarqueur TAI conforme à l'invention permet une meilleure détectabilité de la prostate comme l'atteste la figure 8B. Les CNR calculés à partir d'une carte paramétrique PCb en lien avec le biomarqueur TAI sont plus de deux fois plus grands que ceux obtenus à partir des cartes paramétriques PCc et PCd en lien avec les biomarqueurs ADC et D IVIM connus, notamment à cause d'un niveau de sensibilité au bruit plus important pour ces deux derniers biomarqueurs.

Cette comparaison permet de souligner que l'invention procure un procédé de quantification rapide d'un nouveau biomarqueur de l'activité tissulaire particulièrement résistant et stable aux bruits présents dans les signaux d'imagerie médicale au regard des autres biomarqueurs applicables dans ce contexte. L'invention prévoit ainsi un nouveau biomarqueur exploitable et pertinent dans un grand nombre d'applications parmi lesquelles nous pouvons citer, de manière non exhaustive, l'analyse et/ou le suivi de cancers, l'évaluation d'accidents vasculaires cérébraux.

**Revendications**

1. Procédé (100) pour quantifier un biomarqueur d'un volume élémentaire dit « voxel » d'un organe, ledit procédé étant mis en œuvre par une unité de traitement (4) d'un système d'analyse d'imagerie d'IRM de diffusion et comportant une étape (130) pour produire la valeur dudit biomarqueur, ci-après désigné « indicateur d'activité tissulaire » ou TAI, à partir de données (10, 12) expérimentales S(b) résultant d'une acquisition d'un signal par imagerie d'IRM de diffusion, le procédé (100) étant **caractérisé en ce que** ladite étape (130) pour produire la valeur dudit biomarqueur TAI consiste, sur un intervalle borné, $b_{min}$ à $b_{max}$ de valeurs d'un paramètre d'acquisition b correspondant à l'intensité du gradient de diffusion, en le calcul $TAI = \int_{b_{min}}^{b_{max}} L(b) - \Gamma_S[S(b)]\, db$, L(b) étant une fonction dudit paramètre d'acquisition b et $\Gamma_S[S(b)]$ une transformation bijective desdites données expérimentales S(b).

2. Procédé (100) selon la revendication précédente, pour lequel lesdites fonction et transformation bijective sont mutuellement déterminées, de sorte que L(b) soit supérieure ou égale à $\Gamma_S[S(b)]$, sur l'ensemble des valeurs du paramètre d'acquisition b comprises entre $b_{min}$ et $b_{max}$.

3. Procédé (100) selon la revendication 1 ou 2, pour lequel lesdites fonction et transformation bijective sont mutuellement déterminées, de sorte que $L(b_{min}) = \Gamma_S[S(b_{min})]$ et/ou $L(b_{max}) = \Gamma_S[S(b_{max})]$.

4. Procédé (100) selon l'une quelconque des revendications précédentes, le système d'analyse d'imagerie comportant une interface homme-machine de sortie (5) du biomarqueur (TAI) quantifié à un utilisateur (6) dudit système, ladite interface homme-machine de sortie (5) coopérant avec l'unité de traitement (4), ledit procédé comportant une étape subséquente (140) pour déclencher une sortie dudit biomarqueur quantifié (TAI) selon un format approprié.

5. Procédé (100) selon l'une quelconque des revendications précédentes, le système d'analyse d'imagerie comportant une interface homme-machine d'entrée (8) coopérant avec l'unité de traitement (4), ledit procédé (100) comportant une étape (110) de détermination de la fonction L(b) dudit paramètre d'acquisition b et de la transformation bijective $\Gamma_S$ [S(b)] desdites données expérimentales S(b) à partir de données d'entrée d'un utilisateur de ladite interface homme-machine d'entrée (8).

6. Procédé (100) selon l'une quelconque des revendications précédentes, pour lequel l'étape (130) pour produire la valeur dudit biomarqueur est mise en œuvre par itérations successives pour une pluralité de voxels considérés, ledit biomarqueur (TAI) étant quantifié par voxel.

7. Procédé (100) selon les revendications 6 et 4, l'étape (140) pour déclencher une sortie dudit biomarqueur (TAI) quantifié consiste à générer une image sous la forme d'une carte paramétrique dont les pixels encodent respectivement les valeurs dudit biomarqueur quantifié pour les voxels considérés.

8. Système d'analyse d'imagerie comportant une unité de traitement (4), des moyens pour communiquer avec le monde extérieur et des moyens de mémorisation, **caractérisé en ce que** :

   - les moyens pour communiquer sont agencés pour recevoir du monde extérieur des données expérimentales S(b) d'un volume élémentaire d'un organe ;
   - les moyens de mémorisation comportent des instructions dont l'interprétation ou l'exécution par ladite unité de traitement provoque la mise en œuvre d'un procédé (100) pour quantifier un biomarqueur d'un volume élémentaire dudit organe selon l'une quelconque des revendications 1 à 7.

9. Système d'analyse d'imagerie selon la revendication précédente, pour lequel :

   - les données expérimentales S(b) d'un volume élémentaire d'un organe sont des données résultant d'une acquisition d'un signal par imagerie d'IRM de diffusion ;
   - le biomarqueur quantifié est un indicateur de la diffusion de molécules d'eau (TAI) dans un volume élémentaire dudit organe.

10. Système selon la revendication 8 ou 9, pour lequel les moyens pour communiquer sont agencés pour transmettre un contenu graphique, en lien avec ledit biomarqueur quantifié par la mise en œuvre dudit procédé (100) selon la revendication 4, à une interface homme-machine de sortie (5).

11. Système selon l'une quelconque des revendications 8 à 10, pour lequel les moyens pour communiquer sont agencés pour recueillir des données d'entrée transmises par une interface homme-machine d'entrée (2) dudit système, lesdites données d'entrée permettant de déterminer une fonction L(b) du paramètre d'acquisition b et une transformation bijective $\Gamma_S[S(b)]$ desdites données expérimentales S(b).

12. Produit programme d'ordinateur comportant une ou plusieurs instructions interprétables ou exécutables par l'unité de traitement (4) d'un système d'analyse d'imagerie conforme à l'une quelconque des revendications 8 à 11, ledit programme étant chargeable dans des moyens de mémorisation dudit système, **caractérisé en ce que** l'interprétation ou l'exécution desdites instructions par ladite unité de traitement provoque la mise en œuvre d'un procédé (100) pour quantifier un biomarqueur (TAI) d'un volume élémentaire selon l'une quelconque des revendications 1 à 7.

**Patentansprüche**

1. Verfahren (100) zum Quantifizieren eines Biomarkers eines "Voxel" genannten Elementarvolumens eines Organs, wobei das Verfahren von einer Verarbeitungseinheit (4) eines Analysesystems für die Diffusions-MRT-Bildgebung implementiert wird und einen Schritt (130) zum Erzeugen des Werts des Biomarkers, nachstehend als "Gewebeaktivitätsindikator" oder TAI (en: Tissue Activitity Indicator) bezeichnet, aus experimentellen Daten S(b) (10, 12), die aus einer Signalerfassung durch Diffusions-MRT-Bildgebung resultieren, umfasst, wobei das Verfahren (100) **dadurch gekennzeichnet ist, dass** der Schritt (130) zum Erzeugen des Werts des Biomarkers TAI darin besteht, über ein beschränktes Intervall $b_{min}$ bis $b_{max}$ von Werten eines Erfassungsparameters b, der der Stärke des Diffusionsgradienten entspricht, $TAI = \int_{b_{min}}^{b_{max}} L(b) - \Gamma_S[S(b)]db$ zu berechnen, wobei L(b) eine Funktion des Erfassungsparameters b und $\Gamma_S[S(b)]$ eine bijektive Transformation der experimentellen Daten S(b) ist.

2. Verfahren (100) nach dem vorstehenden Anspruch, wobei die Funktion und die bijektive Transformation so aufeinander abgestimmt werden, dass L(b) über der Gesamtheit der Werte des Erfassungsparameters b, die zwischen $b_{min}$ und $b_{max}$ liegen, größer oder gleich $\Gamma_S[S(b)]$ ist.

3. Verfahren (100) nach Anspruch 1 oder 2, wobei die Funktion und die bijektive Transformation so aufeinander abgestimmt werden, dass

$$L(b_{min}) = \Gamma_S[S(b_{min})] \text{ und/oder } L(b_{max}) = \Gamma_S[S(b_{max})] \text{ ist.}$$

4. Verfahren (100) nach einem der vorstehenden Ansprüche, wobei das Analysesystem für die Bildgebung eine Mensch-Maschine-Ausgabeschnittstelle (5) des quantifizierten Biomarkers (TAI) an einen Benutzer (6) des Systems aufweist, die Mensch-Maschine-Ausgabeschnittstelle (5) mit der Verarbeitungseinheit (4) zusammenwirkt und das Verfahren einen nachfolgenden Schritt (140) zum Auslösen einer Ausgabe des quantifizierten Biomarkers (TAI) in einem geeigneten Format aufweist.

5. Verfahren (100) nach einem der vorstehenden Ansprüche, wobei das Analysesystem für die Bildgebung eine Mensch-Maschine-Eingabeschnittstelle (8) aufweist, die mit der Verarbeitungseinheit (4) zusammenwirkt, wobei das Verfahren (100) einen Schritt (110) zur Bestimmung der Funktion L(b) des Erfassungsparameters b und der bijektiven Transformation $\Gamma_S[S(b)]$ der experimentellen Daten S(b) aus Eingabedaten eines Benutzers der Mensch-Maschine-Eingabeschnittstelle (8) aufweist.

6. Verfahren (100) nach einem der vorstehenden Ansprüche, wobei der Schritt (130) zum Erzeugen des Werts des Biomarkers durch aufeinanderfolgende Iterationen für eine Vielzahl von betrachteten Voxeln ausgeführt wird, wobei der Biomarker (TAI) je Voxel quantifiziert wird.

7. Verfahren (100) nach den Ansprüchen 6 und 4, wobei der Schritt (140) zum Auslösen einer Ausgabe des quantifizierten Biomarkers (TAI) darin besteht, ein Bild in Form einer parametrischen Karte zu erzeugen, deren Pixel jeweils die Werte des quantifizierten Biomarkers für die betrachteten Voxel codieren.

8. Analysesystem für die Bildgebung mit einer Verarbeitungseinheit (4), Mittel zur Kommunikation mit der Außenwelt und Speichermittel,
   **dadurch gekennzeichnet, dass:**

   - die Kommunikationsmittel dazu eingerichtet sind, von der Außenwelt experimentelle Daten S(b) eines Elementarvolumens eines Organs zu empfangen;
   - die Speichermittel Anweisungen enthalten, deren Interpretation oder Ausführung durch die Verarbeitungseinheit die Durchführung eines Verfahrens (100) zum Quantifizieren eines Biomarkers eines Elementarvolumens des Organs nach einem der Ansprüche 1 bis 7 bewirkt.

9. Analysesystem für die Bildgebung nach dem vorstehenden Anspruch, wobei:

   - die experimentellen Daten S(b) eines Elementarvolumens eines Organs Daten sind, die aus einer Signalerfassung durch Diffusions-MRT-Bildgebung resultieren;
   - der quantifizierte Biomarker ein Indikator für die Diffusion von Wassermolekülen (TAI) in einem Elementarvolumen des Organs ist.

10. System nach Anspruch 8 oder 9, wobei die Kommunikationsmittel dazu eingerichtet sind, einen grafischen Inhalt, der mit dem durch die Durchführung des Verfahrens (100) nach Anspruch 4 quantifizierten Biomarker in Verbindung steht, an eine Mensch-Maschine-Ausgabeschnittstelle (5) zu übertragen.

11. System nach einem der Ansprüche 8 bis 10, wobei die Kommunikationsmittel dazu eingerichtet sind, Eingabedaten zu erfassen, die von einer Mensch-Maschine-Eingabeschnittstelle (2) des Systems übertragen werden, wobei die Eingabedaten das Bestimmen einer Funktion L(b) des Erfassungsparameters b und einer bijektiven Transformation $\Gamma_S[S(b)]$ der experimentellen Daten S(b) ermöglichen.

12. Computerprogrammprodukt mit einer oder mehreren Anweisungen, die von der Verarbeitungseinheit (4) eines Analysesystems für die Bildgebung nach einem der Ansprüche 8 bis 11 interpretierbar oder ausführbar sind, wobei das Programm in Speichermittel des Systems ladbar ist,
**dadurch gekennzeichnet, dass** die Interpretation oder die Ausführung der Anweisungen durch die Verarbeitungseinheit die Durchführung eines Verfahrens (100) zum Quantifizieren eines Biomarkers (TAI) eines Elementarvolumens nach einem der Ansprüche 1 bis 7 bewirkt.

**Claims**

1. Method (100) for quantifying a biomarker of an elementary volume, referred to as a "voxel", of an organ, said method being implemented by a processing unit (4) of a diffusion MRI imaging analysis system and comprising a step (130) for producing the value of said biomarker, hereinafter referred to as a "tissue activity indicator" or TAI, on the basis of data (10, 12) which are experimental data S(b) resulting from an acquisition of a signal by diffusion MRI imaging, the method (100) being **characterized in that** said step (130) for producing the value of said biomarker TAI consists, over a bounded interval $b_{min}$ to $b_{max}$ of values of an acquisition parameter b corresponding to the intensity of the diffusion gradient, in calculating $\mathrm{TAI} = \int_{b_{min}}^{b_{max}} \mathrm{L}(b) - \Gamma s[S(b)]\mathrm{db}$, L(b) being a function of said acquisition parameter b and $\Gamma_S[S(b)]$ being a bijective transformation of said experimental data S(b).

2. Method (100) according to the preceding claim, wherein said function and bijective transformation are mutually determined such that L(b) is greater than or equal to $\Gamma_S[S(b)]$, over all the values of the acquisition parameter b between $b_{min}$ and $b_{max}$.

3. Method (100) according to claim 1 or 2, wherein said function and bijective transformation are mutually determined such that $L(b_{min}) = \Gamma_S[S(b_{min})]$ and/or $L(b_{max}) = \Gamma_S[S(b_{max})]$.

4. Method (100) according to any of the preceding claims, the imaging analysis system comprising an output human-machine interface (5) for outputting the quantified biomarker (TAI) to a user (6) of said system, said output human-machine interface (5) cooperating with the processing unit (4), said method comprising a subsequent step (140) for triggering an output of said quantified biomarker (TAI) in an appropriate format.

5. Method (100) according to any of the preceding claims, the imaging analysis system comprising an input human-machine interface (8) cooperating with the processing unit (4), said method (100) comprising a step (110) of determining the function L(b) of said acquisition parameter b and the bijective transformation $\Gamma_S[S(b)]$ of said experimental data S(b) on the basis of input data from a user of said input human-machine interface (8).

6. Method (100) according to any of the preceding claims, wherein the step (130) for producing the value of said biomarker is implemented by successive iterations for a plurality of voxels in question, said biomarker (TAI) being quantified per voxel.

7. Method (100) according to claims 6 and 4, the step (140) for triggering an output of said quantified biomarker (TAI) consists in generating an image in the form of a parametric map, the pixels of which respectively encode the values of said quantified biomarker for the voxels in question.

8. Imaging analysis system comprising a processing unit (4), means for communicating with the outside world, and memory means, **characterized in that:**

   - the communication means are arranged to receive, from the outside world, experimental data S(b) of an elementary volume of an organ;
   - the memory means comprise instructions, the interpretation or execution of which by said processing unit causes the implementation of a method (100) for quantifying a biomarker of an elementary volume of said organ according to any of claims 1 to 7.

9.  Imaging analysis system according to the preceding claim, wherein:

    - the experimental data S(b) of an elementary volume of an organ are data resulting from an acquisition of a signal by diffusion MRI imaging;
    - the quantified biomarker is an indicator of the diffusion of water molecules (TAI) in an elementary volume of said organ.

10. System according to claim 8 or 9, wherein the communication means are arranged to transmit graphic content, which is linked with said quantified biomarker by the implementation of said method (100) according to claim 4, to an output human-machine interface (5).

11. System according to any of claims 8 to 10, wherein the communication means are arranged to collect input data transmitted by an input human-machine interface (2) of said system, said input data allowing a function L(b) of the acquisition parameter b and a bijective transformation $\Gamma_S[S(b)]$ of said experimental data S(b) to be determined.

12. Computer program product comprising one or more instructions that can be interpreted or executed by the processing unit (4) of an imaging analysis system according to any of claims 8 to 11, said program being loadable into memory means of said system, **characterized in that** the interpretation or execution of said instructions by said processing unit causes the implementation of a method (100) for quantifying a biomarker (TAI) of an elementary volume according to any of claims 1 to 7.

FIG.1

FIG.2

FIG.3

FIG.4

FIG.5

FIG.6A

FIG.6B

FIG.6C

FIG.7B

FIG.7C

FIG.7D

FIG.7A

FIG.8A

FIG.8B

**EP 4 154 215 B1**

## RÉFÉRENCES CITÉES DANS LA DESCRIPTION

**Documents brevets cités dans la description**

- EP 3387457 A1